# EUROPEAN PATENT SPECIFICATION

(11) **EP 1 113 823 B1**
(45) Date of publication and mention of the grant of the patent: **04.08.2004**
(21) Application number: 99969037.3
(22) Date of filing: 15.09.1999
(51) Int. Cl.: A61K 49/04

(54) **CHIRAL RADIO-CONTRAST AGENTS**
CHIRALE KONTRASTMITTEL
AGENTS DE CONTRASTE RADIOLOGIQUE

(30) Priority: 16.09.1998 EP 98117598
(43) Date of publication of application: 11.07.2001
(73) Proprietor: Omnia Pharma S.R.L., 00143 Roma (IT)
(72) Inventor: FRANK, Artur, D-86441 Zusmarshausen (DE); GIANNETTINO, Andreina, I-00143 Roma (IT)
(74) Representative: Gervasi, Gemma, Dr.
(86) International application number: PCT/EP1999/006830
(87) International publication number: WO 2000/015266

(56) References cited:
- WO-A-97/02235
- WO-A-97/19705
- WO-A-98/34908
- FELDER E ET AL: "IOPAMIDOL" 1 January 1988 (1988-01-01) , ANALYTICAL PROFILES OF DRUG SUBSTANCES, VOL. 17, PAGE(S) 115 - 154 XP000562617 the whole document
- DAWSON, PETER ET AL.: "Isomeric Purity and Supersaturation of Iopamidol" BR. J. RADIOL., vol. 56, no. 670, 1983, pages 711-713, XP002096202
- PITRE D. ET AL.: "Development, Chemistry and Physical Properties of Iopamidol and its Analogs" INVESTIGATIVE RADIOLOGY, vol. 15, no. 6 Suppl., 1980, pages S301-S306, XP002096203
- THE MERK LAORATORIES: "The Merk Index" , MERK AND CO. INC. , N.J. (U.S.A.) XP002131835 page 862 page 866-869

## Description

The present invention relates to radio-contrast agents and, in particular, to water soluble, non-ionic x-ray contrast agents or media.

Most known contrast or imaging agents are to some extent pharmacologically active and their use, thus, can cause adverse side effects in patients. These adverse pharmacological effects are often referred to as "chemotoxic" effects. It has been suggested that an agent's chemotoxic effect stems from its capacity to bind protein and its resulting ability to inhibit certain enzyme systems and to interfere with normal metabolic pathways by binding cell surface receptor proteins. For example, certain contrast agents have been shown to cause tachycardia and others to interfere with normal hemostatic processes.

Many non-ionic contrast agents include at least one chiral carbon atom and it is well known that the stereoisomers of optically active compounds can exhibit markedly different pharmacological properties. In particular, it is common for one stereoisomer of a pair to be markedly more active or chemotoxic than the other. For example, the L-form of the beta-adrenergic blocking agent popranolol, is a hundred times more potent than the D-enantiomer. It has also been suggested that the D-enantiomer of thalidomide is both safe and an effective sedative, even when used for the control of morning sickness during pregnancy, while the corresponding L-enantiomer is now believed to have been responsible for the racemic drug's notorious teratogenic effect.

Dawson, P. et al.: "Isomeric Purity and Supersaturation of Iopamidol" Br. J. Radiol., vol. 56, n° 670, 1983, pages 711-713 discloses that the solubility of iopamidol probably depends on the isomeric purity of the preparation, being for instance the solubility of the L(S)-form higher as the racemate's one. A typical D(R)-isomer concentration is 1.6%. The document neither mentions a solution of iopamidol with a concentration of R-iopamidol higher than 50% nor discloses the relationship between R-iopamidol and fewer adverse side effects on administration (chemotoxicity).

The present invention is based upon the understanding that imaging or contrasting agents, ideally, should be pharmacologically inert and stems from a realisation that the above discussed properties of optically active compounds can be exploited in order to bring this ideal closer to realisation.

Accordingly, the present invention provides an imaging or contrast agent comprising a stereoisomer of a compound with at least one chiral centre, wherein said stereoisomer is in stereoisomeric excess and causes fewer adverse side effects on administration, or is less chemotoxic, than at least one other stereoisomer of said chiral compound. A chiral compound is understood to include a stereoisomeric excess of a particular stereoisomer when the latter is present in a greater proportion that it would be in the racemic compound.

Preferably, the compound includes a stereoisomeric excess of a single stereoisomer which causes fewer side effects or is less chemotoxic than at least one other stereoisomer of the compound. More preferably, said single stereoisomer causes the fewest adverse side effects, or is the least chemotoxic, of said chiral compound's stereoisomers.

In preferred embodiments, the stereoisomer in stereoisomeric excess has a lower affinity for a human receptor than at least one other stereoisomer of said chiral compound. Preferably, said single stereoisomer has the lowest affinity for a human receptor of said chiral compound's stereoisomers.

An advantage of contrast agents in accordance with the present invention is that they can be employed in greater quantities or concentrations than their equivalents which are either racemic, or consist of a more chemotoxic stereoisomer.

Said chiral compound need not be optically pure but, in preferred embodiments, greater than 50% thereof is in the form of said single stereoisomer. Preferably, greater than 70, 75, 80, 85, 90, 95, or 98% of said compound is in the form of said single stereo isomer. Said single stereoisomer is preferably in the R form.

The compound is iopamidol and the stereoisomer of this agent which must be in stereoisomeric excess is R-iopamidol. Currently, iopamidol is only available in its more chemotoxic S-stereoisomeric form.

### Example 1

### Preparation of R iopamidol [R-5-(α-hydroxypropionylamino)-2,4,6-triiodoisophthalic acid di-(1,3 dihydroxyisopropylamide)].

400g (0.72 Mole) 5-amino-2,4,6-triiodoisophthalic acid should be added to 200 ml thionyl chloride, the mixture boiled and stirred for six hours, and the resulting solution then evaporated. The residue should then be dissolved in anhydrous ethyl acetate, and the solution should again be evaporated to dryness. The solid material should then be dissolved in 4000 ml ethyl acetate, and the solution stirred into an ice-cold solution of 500 g sodium chloride and 200 g sodium bicarbonate in 2.5 liters water. The organic phase should be separated from the aqueous solution, washed with aqueous sodium chloride solution, dried by contact with anhydrous calcium chloride, and evaporated to dryness.

The resulting 5-amino-2,4,6,-triiodoisophthalyl chloride has a melting point of about 300°C when recrystallized from toluene.

300 g (0.503 mole) 5-amino-2,4,6-triiodoisophthalyl chloride should be dissolved in 1200 ml dimethylacetamide, and 187 g (1.26 mole) R-2 acetoxypropionyl chloride added dropwise to the solution at 3-5°C with agitation. The mixture should be permitted to stand overnight at ambient temperature and then should be evaporated in a vacuum to approximately 400 ml. The oily residue should be stirred into ice water to precipitate crystalline R-5-(α-acetoxypropionylamino) 2,4,6-triiodoisophthalyl chloride which should then be purified by suspension in warm alcohol free chloroform.

28.4 g (0.04 mole) of the purified R-5-(α-acetoxypropionylamino) 2,4,6-triiodoisophthalyl chloride should then be dissolved in 150 ml dimethylacetamide and 15 g (0.08 mole) tributylamine. The mixture should be heated to 50° and 9.1 g (0.01 mole) 1,3-dihydroxyisopropylamine (2-amino-1,3-propanediol) dissolved in 80 ml dimethylacetamide added drop by drop. The reaction should go completion within a few hours, and the reaction mixture should then be evaporated to dryness in a vacuum. The oily residue should be added to 350 ml methylene chloride with vigorous agitation, and the resulting precipitate filtered off and purified by repeated suspension in warm methylene chloride.

The resulting R-5-(α-acetoxypropionylamino)-2,4,6-triiodoisophthalic acid di-(1-3-dihydroxyisopropylamide) should then be dissolved in water and the solution decolourized with active carbon, adjusted to pH 11 with concentrated sodium hydroxide solution, heated to 40°C, and mixed with additional NaOH solution until the pH has stabilized, indicating the complete saponification of the acetoxy groups.

Sequential contact with cation and anion exchange resins will remove the salts from the saponification mixture, and the deionized solution should then be evaporated to dryness. The residue should be further purified by recrystallisation from ethanol to provide the required R-5-(α-hydroxxypropionylamino)-2,4,6-triiodoisophthalic acid di-(1-3-dihydroxyisopropylamide).

### Example 2

### Formulation for injection.

The ingredients set out in the following table were mixed together to provide a contrast agent composition in accordance with the present invention.

R-iopamidol (greater than 70% stereoisomeric purity, preferably greater than 98% stereoisomeric purity).
Ca-EDTA
Tromethamine.
HCl
Water for injection.

## Claims

1. An imaging or contrast agent comprising iopamidol **characterised in that** said agent comprises more than 50 % of R-iopamidol.

2. The imaging or contrast agent as claimed in claim 1, wherein said agent comprises more than 80% R-iopamidol.

3. A Use of R-iopamidol for the manufacture of an imaging or contrast agent according to claim 1 or 2.

4. Use of R-iopamidol according to claim 3 wherein said imaging or contrast agent shows low chemotoxicity and/or causes few adverse side effects on administration.

## Patentansprüche

1. Bildgebungs- oder Kontrastmittel, das Iopamidol umfasst, **dadurch gekennzeichnet, dass** das Mittel mehr als 50 % R-Iopamidol umfasst.

2. Bildgebungs- oder Kontrastmittel gemäss Anspruch 1, worin das Mittel mehr als 80 % R-Iopamidol umfasst.

3. Verwendung von R-Iopamidol zur Herstellung eines Bildgebungs- oder Kontrastmittels gemäss Anspruch 1 oder 2.

4. Verwendung von R-Iopamidol gemäss Anspruch 3, worin das Bildgebungs- oder Kontrastmittel bei der Verabreichung eine geringe Chemotoxizität zeigt und/oder wenige nachteilige Nebenwirkungen hervorruft.

## Revendications

1. Agent d'imagerie ou de contraste comprenant de l'iopamidol **caractérisé en ce que** ledit agent comprend plus de 50% de R-iopamidol.

2. Agent d'imagerie ou de contraste selon la revendication 1, où ledit agent comprend plus de 80% de R-iopamidol.

3. Utilisation de R-iopamidol pour la fabrication d'un agent de contraste ou d'imagerie selon la revendication 1 ou 2.

4. Utilisation du R-iopamidol selon la revendication 3 où ledit agent de contraste ou d'imagerie montre une faible chimio-toxicité et/ou provoque peu d'effets secondaires néfastes lors d'une administration.
